# EUROPEAN PATENT SPECIFICATION

(11) **EP 3 586 911 B1**
(45) Date of publication and mention of the grant of the patent: **03.01.2024**
(21) Application number: 17897519.9
(22) Date of filing: 26.04.2017
(51) Int. Cl.: A61N 1/36, A61B 3/08, A61N 1/372

(54) **IMPLANTABLE EXTRAOCULAR MUSCLE NEUROMUSCULAR STIMULATOR AND PARAMETER SETTING METHOD THEREFOR**
IMPLANTIERBARER NEUROMUSKULÄRER STIMULATOR FÜR EXTRAOKULAREN MUSKEL UND PARAMETEREINSTELLVERFAHREN DAFÜR
STIMULATEUR IMPLANTABLE NEUROMUSCULAIRE DE MUSCLE EXTRA-OCULAIRE ET PROCÉDÉ DE RÉGLAGE DE PARAMÈTRES ASSOCIÉ

(30) Priority: 21.02.2017 CN 201710092691
(43) Date of publication of application: 01.01.2020
(73) Proprietor: Chaomu Technology (Beijing) Co., Ltd., Haidian District Beijing 100080 (CN)
(72) Inventor: WANG, LeJin, Beijing 100044 (CN); MIAO, ZeQun, Beijing 100044 (CN); LU, Yao, Beijing 100044 (CN); MENG, XiaoLi, Beijing 100044 (CN); GUO, LiLi, Beijing 100044 (CN); DONG, JiaMei, Beijing 100044 (CN); LI, LuoJia, Beijing 100044 (CN)
(74) Representative: Cabinet Bleger-Rhein-Poupon
(86) International application number: PCT/CN2017/081942
(87) International publication number: WO 2018/152954

(56) References cited:
- CN-A- 1 745 857
- CN-A- 101 883 607
- CN-A- 103 747 834
- US-A1- 2009 005 756
- US-A1- 2009 005 756
- US-A1- 2009 312 817
- US-A1- 2015 039 057
- US-A1- 2015 224 318
- US-B1- 9 302 103

## Description

### TECHNICAL FIELD

The present application relates to the field of miniature medical equipment, and particularly relates to an implantable extraocular-muscle neuromuscular stimulator and its parameter setting method.

### BACKGROUND

Nystagmus is involuntary, rhythmic and reciprocal eyeball swinging or jumping, and it has complicated clinical manifestation, and is frequently accompanied with visual impairment of both eyes. According to the primary diseases of nystagmus, nystagmus is classified as types such as ocular nystagmus, vetibular nystagmus, central nystagmus and cryptogenic nystagmus.

Among them, ocular nystagmus is clinically closely relevant to ophthalmology, and is mainly congenital nystagmus (CN), which frequently emerges at birth or within 4 months after birth. According to statistics, its prevalence rate among children is 1/1000-1/1500, and globally there are approximately millions of patients. According to the etiologies, it is classified as: Sensory Defect Nystagmus (SDN) and Congenital Idiopathic Nystagmus (CIN).

CIN is congenital defective outgoing-mechanism and normal passing mechanism, and the abnormality of non-closing eyes is frequently expressed as impulsive nystagmus. The main harm of CIN is resulting in amblyopia. According to statistics, among the patients, the cases of illness of amblyopia account for 86.7%, wherein moderate and severe amblyopia account for 20.2%, and moderate amblyopia accounts for 52.3%. Accordingly, it can be seen that, CIN affects vision widespreadly.

Because the particular pathogenesis of CIN has not been clear, and there is no clinically effective approach, the disease is always one of the difficult and key diseases in ophthalmology.

Implantable medical-treatment instruments are diverse, such as cardiac pacemaker, defibrillator and implantable muscle stimulator. The present application relates to an implantable extraocular-muscle neuromuscular stimulator and its parameter setting method for treating congenital idiopathic nystagmus. US Pat. Pub. 2009/005756 relates to generating stimulus to treat strabismus with stimulator, and applying stimulus to stimulation site within patient, where site includes cranial nerve for innervating extraocular muscle.

### SUMMARY OF THE DISCLOSURE

Regarding the above problems, the present application proposes an implantable extraocular-muscle neuromuscular stimulator and its parameter setting method, for treating congenital idiopathic nystagmus. The stimulator, by detecting the contraction state of the extraocular muscle and the stimulating signal, supplies the extraocular muscle the corresponding stimulation and contraction, to balance the nerve stimulation and counteract the contraction, to enable the eyeballs to maintain fixed staring and not to have nystagmus.

The technical solution of the present application:
In order to solve the above technical problem, the present application provides an implantable extraocular-muscle neuromuscular stimulator, comprising an implantable pulse generator, an extracorporeal remote controller and an electrode, wherein the implantable pulse generator is provided in a created subcutaneous pouch, the implantable pulse generator is connected to an extraocular muscle by the electrode, and the implantable pulse generator is for generating an pulse stimulating signal; the electrode comprises a lead and a contact-pin connector and a contact that are provided at two ends of the lead, wherein the contact-pin connector is connected to the implantable pulse generator, the lead is provided in a created subcutaneous tunnel, and the contact is connected to the extraocular muscle; and the extracorporeal remote controller is connected to the implantable pulse generator by a wireless signal, and is for setting the pulse stimulating signal of the implantable pulse generator; wherein the implantable pulse generator is for sensing a contraction state of an eye muscle by using the electrode, and according to the contraction state of the eye muscle generating an eye-muscle stimulating signal, and sending the eye-muscle stimulating signal to the extracorporeal remote controller; and the implantable pulse generator is further for receiving an eye-muscle stimulating signal of an opposite direction that is emitted by the extracorporeal remote controller, and outputting the eye-muscle stimulating signal by using the electrode to another eye muscle. Optionally, the implantable pulse generator comprises a receiver, a transmitter, a wireless energy transfer module, a power management module, a digital processing module and an electrical-stimulation driver, and the wireless energy transfer module transmits energy to the power management module, the receiver and the transmitter; and the implantable pulse generator wirelessly receives the signal by using the receiver, and transmits an output to the digital processing module, the signal is transmitted to the electrical-stimulation driver via a current generating circuit and a waveform time-sequence circuit, and the electrical-stimulation driver is connected to the electrode.

Optionally, the extracorporeal remote controller comprises a receiver, a transmitter, a frequency synthesizer, a digital processing module, a data analysis module, a data storage module, a display and a processor, the processor is connected to the data analysis module, the data storage module, a controlling program and the display, and the frequency synthesizer is connected to the digital processing module, the receiver and the transmitter; the extracorporeal remote controller wirelessly receives the signal by using the receiver, and transmits the signal to the digital processing module, and the signal is transmitted to the processor via a digital interface circuit; and the processor transmits the signal to the data analysis module, and stores an analysis result to the data storage module, and the processor transmits the analysis result to the implantable pulse generator via the digital interface circuit, the digital processing module and the transmitter.

Optionally, the contact on the electrode is a plurality of contacts, and when the contacts are connected to the extraocular muscle, at least one of the contacts is connected to the extraocular muscle; and the contact is connected to the extraocular muscle by binding or insertion.

Optionally, the setting of the pulse stimulating signal comprises: pulse amplitude setting, pulse width setting and pulse frequency setting.

Optionally, the pulse amplitude setting comprises a voltage mode and a current mode, wherein when a pulse amplitude is set in the voltage mode, a voltage range is 0-10V, and an adjustment step length is 1V or 0.1V; and when a pulse amplitude is set in the current mode, a current range is 0-25mA, and an adjustment step length is 1mA or 0.1mA.

Optionally, a range of the pulse width setting is 30-450ms, and an adjustment step length is 10ms.

Optionally, a range of the pulse frequency setting is 1-500Hz.

The present application further discloses a method for setting a parameter of the implantable extraocular-muscle neuromuscular stimulator, wherein the setting the pulse stimulating signal of the implantable pulse generator by using the extracorporeal remote controller comprises: pulse amplitude setting, pulse width setting and pulse frequency setting.

Optionally, the pulse amplitude setting comprises a voltage mode and a current mode, wherein when a pulse amplitude is set in the voltage mode, a voltage range is 0-10V, and an adjustment step length is 1V or 0.1V; and when a pulse amplitude is set in the current mode, a current range is 0-25mA, and an adjustment step length is 1mA or 0.1mA; a range of the pulse width setting is 30-450ms, and an adjustment step length is 10ms; and a range of the pulse frequency setting is 1-500Hz.

The advantageous effects of the present application:
The implantable extraocular-muscle neuromuscular stimulator and its parameter setting method provided by the present application are applied to the treatment of congenital idiopathic nystagmus. The stimulator, by detecting the contraction state of the extraocular muscle and the stimulating signal, supplies the extraocular muscle the corresponding stimulation and contraction, to balance the nerve stimulation and counteract the contraction, to enable the eyeballs to maintain fixed staring and not to have nystagmus.

### BRIEF DESCRIPTION OF THE DRAWINGS

By the following detailed description made by referring to the drawings, the above and/or other aspects and advantages of the present application will become clearer and easier to understand. The drawings are merely illustrative, and do not limit the present application. In the drawings:
Fig. 1 is a schematic structural diagram of the present application;
Fig. 2 is a module diagram of the implantable pulse generator of the present application;
Fig. 3 is a module diagram of the extracorporeal remote controller of the present application;
Fig. 4 is a schematic structural diagram of the electrode of the present application; and
Fig. 5 is a schematic diagram of the application in nystagmus of the present application.

In the drawings, the reference numbers indicate the following components:
1. implantable pulse generator; 2. extracorporeal remote controller; 3. electrode; 4. extraocular muscle; 5. contact-pin connector; 6. contact; and 7. lead.

### DETAILED DESCRIPTION OF THE DISCLOSURE

The implantable extraocular-muscle neuromuscular stimulator and its parameter setting method of the present application are described in detail below by referring to the particular embodiments and the drawings.

The embodiments set forth herein are particular embodiments of the present application, are used to explain the concept of the present application, are all explanatory and exemplary, and should not be interpreted as limiting the embodiments of the present application and the scope of the present application. Besides the embodiments set forth herein, a person skilled in the art can employ other apparent technical solutions on the basis of the contents disclosed by the claims and the description of the present application, and those technical solutions include technical solutions that make any apparent substitutions and amendments by using the embodiments set forth herein.

The drawings of the description are schematic diagrams, to auxiliarily explain the concept of the present application, and schematically illustrate the shapes of and the relations between the parts. It should be noted that, in order to facilitate clarity illustrating the structures of the components of the embodiments of the present application, the drawings are not drawn in the same proportion. The same reference numbers indicate the same elements.

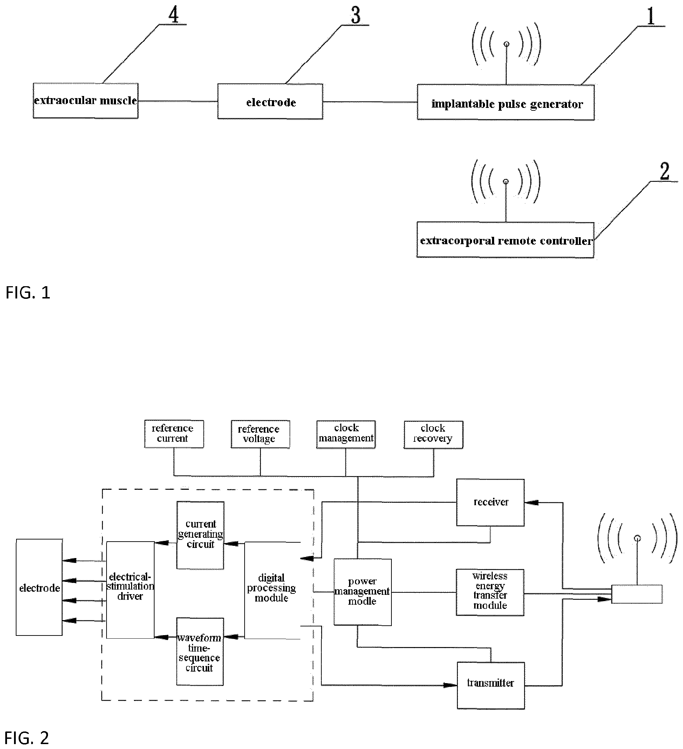

Fig. 1 shows a schematic structural diagram of the implantable extraocular-muscle neuromuscular stimulator. It comprises an implantable pulse generator 1, an extracorporeal remote controller 2 and an electrode 3. The implantable pulse generator 1 is provided in a created subcutaneous pouch, the implantable pulse generator 1 is connected to an extraocular muscle 4 by the electrode 3, and the implantable pulse generator 1 is for generating an pulse stimulating signal.

The extracorporeal remote controller 2 is connected to the implantable pulse generator 1 by a wireless signal, and is for setting the pulse stimulating signal of the implantable pulse generator 1.

Fig. 2 is a module diagram of the implantable pulse generator of the present application. It comprises a receiver, a transmitter, a wireless energy transfer module, a power management module, a digital processing module and an electrical-stimulation driver, and the wireless energy transfer module transmits energy to the power management module, the receiver and the transmitter; and the implantable pulse generator 1 wirelessly receives the signal by using the receiver, and transmits an output to the digital processing module, the signal is transmitted to the electrical-stimulation driver via a current generating circuit and a waveform time-sequence circuit, and the electrical-stimulation driver is connected to the electrode 3.

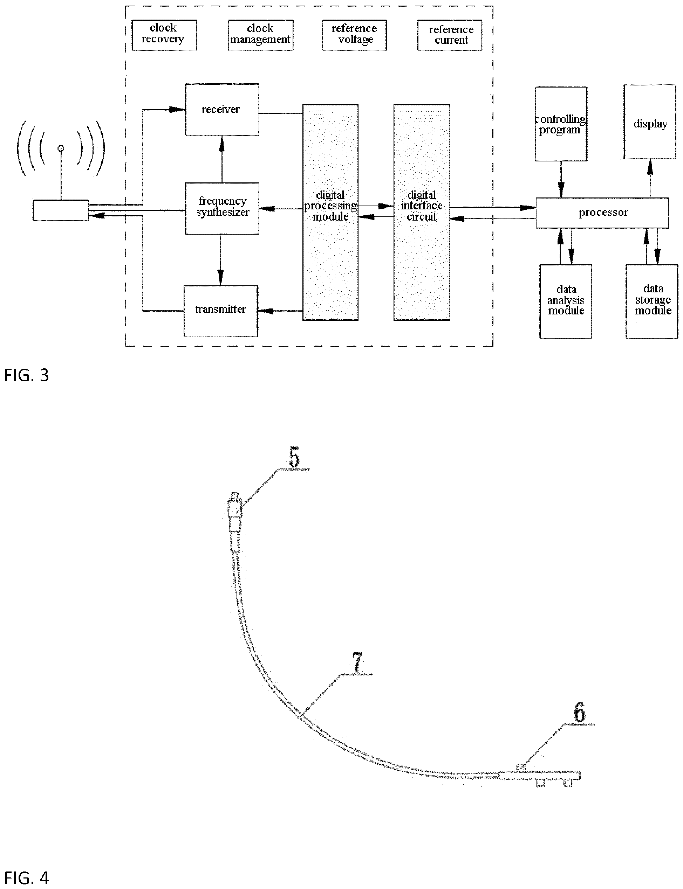

Fig. 3 is a module diagram of the extracorporeal remote controller of the present application. The extracorporeal remote controller 2 comprises a receiver, a transmitter, a frequency synthesizer, a digital processing module, a data analysis module, a data storage module, a display and a processor, the processor is connected to the data analysis module, the data storage module, a controlling program and the display, and the frequency synthesizer is connected to the digital processing module, the receiver and the transmitter; the extracorporeal remote controller 2 wirelessly receives the signal by using the receiver, and transmits the signal to the digital processing module, and the signal is transmitted to the processor via a digital interface circuit; and the processor transmits the signal to the data analysis module, and stores an analysis result to the data storage module, and the processor transmits the analysis result to the implantable pulse generator 1 via the digital interface circuit, the digital processing module and the transmitter.

Fig. 4 is a schematic structural diagram of the electrode of the present application. The electrode 3 comprises a lead 7 and a contact-pin connector 5 and a contact 6 that are provided at two ends of the lead 7, wherein the contact-pin connector 5 is connected to a top cover of the implantable pulse generator 1, the lead 7 is provided in a created subcutaneous tunnel, and the contact 6 is connected to the extraocular muscle 4.

The contact 6 on the electrode 3 is a plurality of contacts, and when the contacts 6 are connected to the extraocular muscle 4, at least one of the contacts 6 is connected to the extraocular muscle 4; and the contact 6 is connected to the extraocular muscle 4 by binding or insertion.

The lead 7 is also referred to as an electrode main body, and the length of the lead is generally 20cm-60cm.

The present application further provides a method for setting a parameter of the implantable extraocular-muscle neuromuscular stimulator, wherein the setting the pulse stimulating signal by using the extracorporeal remote controller 2 comprises: pulse amplitude setting, pulse width setting and pulse frequency setting.

The pulse amplitude setting comprises a voltage mode and a current mode, wherein when a pulse amplitude is set in the voltage mode, a voltage range is 0-10V, and an adjustment step length is 1V or 0.1V; and when a pulse amplitude is set in the current mode, a current range is 0-25mA, and an adjustment step length is 1mA or 0.1mA.

A range of the pulse width setting is 30-450ms, and an adjustment step length is 10ms; and a range of the pulse frequency setting is 1-500Hz.

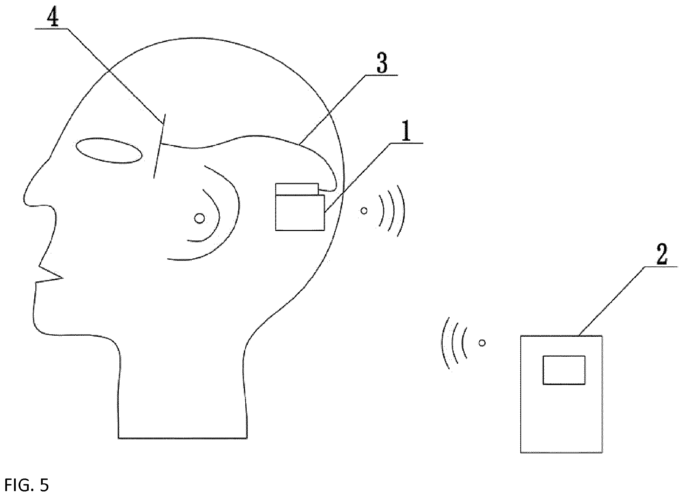

Fig. 5 is a schematic diagram of the application in nystagmus of the present application. The application comprises providing the implantable pulse generator 1 in a subcutaneous pouch behind an ear, binding or inserting the electrode 3 on a peeled extraocular muscle, adjusting the direction of the electrode 3, to avoid the pulling and stress of the electrode 3, and fixing the electrode 3 on the sclera by stitching.

The particular method for treating nystagmus by using the implantable extraocular-muscle neuromuscular stimulator is as follows:
Firstly, when an eyeball of a patient will involuntarily inwardly rotate or outwardly rotate, one of the eye muscles contracts, the implantable pulse generator 1 senses the state of the contraction of that eye muscle by using the electrode 3, analyzes by using the digital processing module on the implantable pulse generator 1 to obtain the degree of the contraction of that eye muscle, and emits an eye-muscle stimulating signal by using the transmitter.

Subsequently, the receiver on the extracorporeal remote controller 2 receives the eye-muscle stimulating signal wirelessly, the digital processing module processes the signal, and transmits to the processor, the processor analyzes the eye-muscle stimulating signal by using the data analysis module, and the transmitter on the extracorporeal remote controller 2 transmits an opposite stimulating signal.

Subsequently, the implantable pulse generator 1 receives the opposite stimulating signal transmitted by the extracorporeal remote controller 2 by using the receiver, and the digital processing module analyzes the signal, and outputs the magnitude and the direction of the stimulating signal that is supplied to the eye muscle.

Subsequently, the electrical-stimulation driver is started up, and the electrode 3 connected to the electrical-stimulation driver supplies the other eye muscle a stimulating signal of the same magnitude and an opposite direction, to cause the other eye muscle to move in the opposite direction and at the same time contract, whereby the forces of the two eye muscles are counteracted, and the eyeball maintains fixed, and does not have nystagmus. Accordingly, the method balances the stimulation on the eye muscles, counteracts the contraction of the eye muscles, and reaches the purpose of treating nystagmus.

When the method is used to treat nystagmus, when an eyeball of a patient will involuntarily inwardly rotate, the medial rectus contracts, the implantable pulse generator 1 senses a contraction signal of the medial rectus, and simultaneously the implantable pulse generator 1 supplies the lateral rectus a stimulating signal of the same magnitude, to cause the lateral rectus to simultaneously contract, whereby the forces of the medial rectus and the lateral rectus are counteracted, and the eyeball maintains fixed, and does not have nystagmus.

In the similar way, when an eyeball of a patient will involuntarily outwardly rotate, the lateral rectus contracts, the implantable pulse generator 1 senses a contraction signal of the lateral rectus, and simultaneously the implantable pulse generator 1 supplies the medial rectus a stimulating signal of the same magnitude, to cause the medial rectus to simultaneously contract, whereby the forces of the lateral rectus and the medial rectus are counteracted, and the eyeball maintains fixed, and does not have nystagmus.

The steps of the surgical method for treating congenital idiopathic nystagmus by using the implantable extraocular-muscle neuromuscular stimulator of the present application are as follows:
S1, peeling to expose the extraocular muscle 4;
S2, binding or inserting the electrode 3 on a peeled extraocular muscle 4;
S3, adjusting the direction of the electrode 3, to avoid the pulling and stress of the electrode 3, and fixing a section of the electrode 3 on the sclera by stitching;
S4, creating a subcutaneous pouch behind an ear for the implantable pulse generator 1;
S5, introducing the electrode 3 via the orbit tunnel and the subcutaneous tunnel to the subcutaneous pouch where the implantable pulse generator 1 is placed;
S6, connecting the implantable pulse generator 1 and the electrode 3, by ensuring that the contact-pin connector 5 on the electrode 3 is inserted into the top cover of the implantable pulse generator 1, and screwing up a fastening screw on the top cover of the implantable pulse generator 1;
S7, performing system test to the implantable pulse generator 1 by using the extracorporeal remote controller 2, to confirm that the system is functioning normally;
S8, encasing the implantable pulse generator 1 into the subcutaneous pouch, and winding the redundant lead around the implantable pulse generator 1;
S9, stitching a stitching hole on the implantable pulse generator 1 and the subcutaneous tissue together, and fixing the implantable pulse generator 1 at the position of the subcutaneous pouch;
S10, performing system test to the implantable pulse generator 1 by using the extracorporeal remote controller 2 again, to confirm that the system is functioning normally; and
S11, disinfecting, and stitching the surgical incision.

By the above operation steps, the stimulator, by detecting the contraction state of the extraocular muscle and the stimulating signal, supplies the extraocular muscle the corresponding stimulation and contraction, to balance the nerve stimulation and counteract the contraction, to enable the eyeballs to maintain fixed staring and not to have nystagmus, which effectively treats the disease of congenital idiopathic nystagmus.

The implantable extraocular-muscle neuromuscular stimulator and its parameter setting method provided by the present application are applied to the treatment of congenital idiopathic nystagmus. The stimulator, by detecting the contraction state of the extraocular muscle and the stimulating signal, supplies the extraocular muscle the corresponding stimulation and contraction, to balance the nerve stimulation and counteract the contraction, to enable the eyeballs to maintain fixed staring and not to have nystagmus.

The present application is not limited to the above embodiments. A person skilled in the art can obtain other technical solutions of various forms by the motivation of the present application. However, regardless of any variation in their shapes or structures, any technical solutions that are the same as or similar to the present application fall within the protection scope of the present application.

## Claims

1. An implantable extraocular-muscle neuromuscular stimulator, comprising an implantable pulse generator (1), an extracorporeal remote controller (2) and an electrode (3), wherein
the implantable pulse generator (1) comprises a receiver, a transmitter, a wireless energy transfer module, a power management module, a digital processing module, and an electrical-stimulation driver, and
the extracorporeal remote controller (2) comprises a receiver, a transmitter, a frequency synthesizer, a digital processing module, a data analysis module, a data storage module, a display and a processor;
the processor is configured to connect to the data analysis module, the data storage module, a controlling program and the display;
the frequency synthesizer is configured to connect to the digital processing module, the receiver and the transmitter;
the extracorporeal remote controller (2) is configured to wirelessly receive the signal by using the receiver, and transmit the signal to the digital processing module; the signal is transmitted to the processor via a digital interface circuit; and
the processor is configured to transmit the signal to the data analysis module, and store an analysis result to the data storage module, and the processor is configured to transmit the analysis result to the implantable pulse generator (1) via the digital interface circuit, the digital processing module and the transmitter;
the wireless energy transfer module is configured to transmit energy to the power management module, the receiver and the transmitter;
the implantable pulse generator (1) is configured to wirelessly receive the signal by using the receiver, and transmit an output to the digital processing module; the signal is transmitted to the electrical-stimulation driver via a current generating circuit and a waveform time-sequence circuit; and the electrical-stimulation driver is configured to connect to the electrode (3);
the implantable pulse generator (1) is configured to be provided in a created subcutaneous pouch, the implantable pulse generator (1) is configured to be connected to an extraocular muscle (4) by the electrode (3), and the implantable pulse generator (1) is for generating *a* pulse stimulating signal;
the electrode (3) comprises a lead (7), a contact-pin connector (5), and a contact (6); wherein the contact-pin connector (5) and the contract (6) are provided at two ends of the lead (7); the contact-pin connector (5) is connected to the implantable pulse generator (1), the lead (7) is configured to be provided in a created subcutaneous tunnel, and the contact (6) is configured to be connected to the extraocular muscle (4); and
the extracorporeal remote controller (2) is connected to the implantable pulse generator (1) by a wireless signal, and is for setting the pulse stimulating signal of the implantable pulse generator (1);
wherein the receiver of the implantable pulse generator (1) is capable of sensing a contraction state of an eye muscle by using the electrode (3), and the digital processing module of the implantable pulse generator (1) is capable of according to the contraction state of the eye muscle generating a first eye-muscle stimulating signal which has one specific magnitude and direction as the contraction state of the eye-muscle and may cause one eye-muscle to move in one specific direction, and the wireless energy transfer module of the implantable pulse generator (1) is capable of sending the first eye-muscle stimulating signal to the extracorporeal remote controller (2);
the processor of the extracorporeal remote controller (2) is programmed to analyzes the first eye-muscle stimulating signal and transmits a second eye-muscle stimulating signal which has the same magnitude and the opposite direction of the first eye-muscle stimulating signal;
the digital processing module of the implantable pulse generator (1) is further capable of receiving the second eye-muscle stimulating signal hat is emitted by the extracorporeal remote controller (2), and outputting the second eye-muscle stimulating signal by using the electrode (3) to another eye muscle;
the second eye-muscle stimulating signal is a stimulation signal that is configured to cause the other eye muscle to move in opposite direction and let eyeball maintains fixed.

2. The implantable extraocular-muscle neuromuscular stimulator according to claim 1, wherein the contact (6) on the electrode (3) is a plurality of contacts, and when the contacts (6) are configured to be connected to the extraocular muscle, at least one of the contacts (6) is adapted to be connected to the extraocular muscle (4); and the contact (6) is adapted to be connected to the extraocular muscle (4) by binding or insertion.

3. The implantable extraocular-muscle neuromuscular stimulator according to claim 1, wherein the setting of the pulse stimulating signal comprises: pulse amplitude setting, pulse width setting and pulse frequency setting.

4. The implantable extraocular-muscle neuromuscular stimulator according to claim 3, wherein the pulse amplitude setting comprises a voltage mode and a current mode, wherein when a pulse amplitude is set in the voltage mode, a voltage range is 0-10V, and an adjustment step length is 1V or 0.1V; and when a pulse amplitude is set in the current mode, a current range is 0-25 mA, and an adjustment step length is 1 mA or 0.1 mA.

5. The implantable extraocular-muscle neuromuscular stimulator according to claim 3, wherein a range of the pulse width setting is 30-450 ms, and an adjustment step length is 10 ms.

6. The implantable extraocular-muscle neuromuscular stimulator according to claim 3, wherein a range of the pulse frequency setting is 1-500 Hz.

7. A method for setting a parameter of the implantable extraocular-muscle neuromuscular stimulator according to claim 1-6, wherein
the setting the pulse stimulating signal of the implantable pulse generator (1) by using the extracorporeal remote controller (2) comprises: pulse amplitude setting, pulse width setting and pulse frequency setting.

8. The method for setting a parameter of the implantable extraocular-muscle neuromuscular stimulator according to claim 7, wherein
the pulse amplitude setting comprises a voltage mode and a current mode, wherein when a pulse amplitude is set in the voltage mode, a voltage range is 0-10V, and an adjustment step length is 1V or 0.1V; and when a pulse amplitude is set in the current mode, a current range is 0-25 mA, and an adjustment step length is 1 mA or 0.1 mA; a range of the pulse width setting is 30-450 ms, and an adjustment step length is 10 ms; and a range of the pulse frequency setting is 1-500 Hz.

## Patentansprüche

1. Ein implantierbarer neuromuskulärer Stimulator für den extraokularen Muskel, umfassend: einen implantierbaren Impulsgeber (1), einen extrakorporale Programmierer (2) und Elektroden (3), **dadurch gekennzeichnet, dass**
der implantierbare Impulsgeber (1) umfassed: einen Empfänger, einen Sender, ein Modul für die drahtlose Energieübertragung, ein Energieverwaltungsmodul, ein digitales Verarbeitungsmodul und einen Treiber für die elektrische Stimulation;
der extrakorporale Programmierer (2) umfassend: Empfänger, Sender, Frequenzsynthesizer, digitale Verarbeitungsmodule, Datenanalysemodule, Datenspeichermodule, Monitor und Prozessoren;
der Prozessor mit Datenanalysemodul, Datenspeichermodul, Steuerprogramm und Monitor verbunden ist;
der Frequenzsynthesizer mit einem digitalen Verarbeitungsmodul, einem Empfänger und einem Sender verbunden ist;
der extrakorporale Programmierer (2) Signale drahtlos über einen Empfänger empfängt und sie an ein digitales Verarbeitungsmodul sendet, wo die Signale über eine digitale Schnittstellenschaltung an einen Prozessor übertragen werden;
der Prozessor das Signal an ein Datenanalysemodul überträgt und die Ergebnisse der Analyse in einem Datenspeichermodul speichert, der Prozessor die Ergebnisse der Analyse an den implantierbaren Impulsgeber (1) über eine digitale Schnittstellenschaltung, ein digitales Verarbeitungsmodul, einen Sender überträgt;
das Modul für die drahtlose Energieübertragung Energie für das Energiemanagementmodul, den Empfänger und den Sender überträgt;
der implantierbare Impulsgeber (1) in der geschaffenen subkutanen Tasche vorgesehen ist, und der implantierbare Impulsgeber (1) über Elektroden (3) mit den extraokularen Muskeln (4) verbunden ist, und der implantierbare Impulsgeber (1) zur Erzeugung eines Impulsstimulationssignals verwendet wird;
die Elektrode (3) einen Draht (7) und jeweils an jedem Ende des Drahtes angeordnet Stiftverbinder (5) und Kontakt (6) umfasst, wobei der Stiftverbinder (5) mit dem implantierbaren Impulsgeber (1) verbunden ist, der Draht (7) in einem geschaffenen subkutanen Tunnel angeordnet ist und der Kontakt (6) mit dem extraokularen Muskel (4) verbunden ist;
der extrakorporale Programmierer (2) drahtlos mit dem implantierbaren Impulsgeber (1) verbunden ist, um das Impulsstimulationssignal für den implantierbaren Impulsgeber (1) einzustellen;
wobei der implantierbare Impulsgeber (1) verwendet wird, um eine Kontraktion eines extraokularen Muskels mittels einer Elektrode (3) zu erfassen, und das digitale Verarbeitungsmodul auf dem implantierbaren Impulsgeber (1) verwendet wird, um ein erstes extraokulares Muskelstimulationssignal basierend auf dem Grad der Kontraktion des extraokularen Muskels und über einen Sender zu übertragen; die Amplitude und die Richtung des ersten extraokularen Muskelstimulationssignals die gleichen wie die Amplitude und die Richtung des Kontraktionsgrades der extraokularen Muskeln sind; das Modul für die drahtlose Energieübertragung verwendet wird, um das erste extraokulare Muskelstimulationssignal an den extrakorporale Programmierer (2) auszugeben;
der extrakorporale Programmierer (2) auch dazu dient, das erste extraokulare Muskelstimulationssignal zu analysieren und ein zweites extraokulares Muskelstimulationssignal mit derselben Amplitude und in entgegengesetzter Richtung zum ersten extraokularen Muskelstimulationssignal abzugeben;
das digitale Verarbeitungsmodul des implantierbaren Impulsgebers (1) auch dazu dient, das zweite extraokulare Muskelstimulationssignal zu empfangen, das von dem extrakorporalen Programmierer ausgegeben wird, und das zweite extraokulare Muskelstimulationssignal an den anderen extraokularen Muskel auszugeben;
das zweite Stimulierungssignal des extraokularen Muskels ein Stimulierungssignal ist, das den anderen extraokularen Muskel veranlasst, sich in der entgegengesetzten Richtung zusammenzuziehen, um das Auge fixiert zu halten.

2. Implantierbarer neuromuskulärer Stimulator nach Anspruch 1, **dadurch gekennzeichnet, dass** die Elektrode (3) eine Vielzahl von Kontakten (6) aufweist, und wenn mindestens einer der Kontakte (6) eine Verbindung mit dem extraokularen Muskel (4) herstellt, sind mindestens ein Kontakt (6) mit dem extraokularen Muskel (4) verbunden; die Kontakte (6) mit dem extraokularen Muskel (4) so verbunden sind, dass sie gebündelt oder eingesetzt sind.

3. Implantierbarer neuromuskulärer Stimulator nach Anspruch 1, **dadurch gekennzeichnet, dass** die besagten Einstellungen des Impulsstimulationssignals die Einstellung der Impulsamplitude, der Impulsbreite und der Impulsfrequenz umfasst.

4. Implantierbarer neuromuskulärer Stimulator nach Anspruch 3, **dadurch gekennzeichnet, dass** die Einstellung der Impulsamplitude einen Spannungsmodus und einen Strommodus umfasst; beim Einstellen der Impulsamplitude im Spannungsmodus der Spannungsbereich 0-10 V beträgt, und der Einstellschritt 1 V oder 0,1 V ist; beim Einstellen der Impulsamplitude im Strommodus der Strombereich 0-25 mA beträgt, und der Einstellschritt 1 mA oder 0,1 mA ist.

5. Implantierbarer neuromuskulärer Stimulator nach Anspruch 3, **dadurch gekennzeichnet, dass** der Einstellungsbereich der Impulsbreite 30-450ms beträgt mit einem Einstellschritt von 10ms.

6. Implantierbarer neuromuskulärer Stimulator nach Anspruch 3, **dadurch gekennzeichnet, dass** der Einstellungsbereich der besagten Impulsfrequenz 1-500Hz beträgt.

7. Verfahren zur Einstellung der Parameter eines implantierbaren neuromuskulären Stimulators für den extraokularen Muskel nach einem der Ansprüche 1-6, **dadurch gekennzeichnet, dass** die Einstellung von Pulsstimulationssignalen für den implantierbaren Impulsgeber (1) mittels eines extrakorporalen Programmierers (2), einschließlich: Einstellung der Impulsamplitude, der Impulsbreite und der Impulsfrequenz.

8. Verfahren zur Einstellung der Parameter eines implantierbaren neuromuskulären Stimulators für den extraokularen Muskel nach Anspruch 7, **dadurch gekennzeichnet, dass** die Einstellung der Impulsamplitude einen Spannungsmodus und einen Strommodus umfasst; beim Einstellen der Impulsamplitude im Spannungsmodus der Spannungsbe-reich 0-10 V beträgt, und der Einstellschritt 1 V oder 0,1 V ist; beim Einstellen der Impulsamplitude im Strommodus der Strombereich 0-25 mA beträgt, und der Einstellschritt 1 mA oder 0,1 mA ist;
der Einstellungsbereich der Impulsbreite 30-450ms beträgt mit einem Einstellschritt von 10ms;
der Einstellungsbereich der besagten Impulsfrequenz 1-500Hz beträgt.

## Revendications

1. Stimulateur neuromusculaire extraoculaire implantable, comprenant un générateur d'impulsion implantable (1), un contrôleur programmable extracorporel (2) et une électrode (3), **caractérisé en ce que**,
le générateur d'impulsion implantable (1) comprend un récepteur, un émetteur, un module de transmission d'énergie sans fil, un module de gestion d'alimentation, un module de traitement numérique et un mécanisme d'entraînement de stimulation électrique ;
le contrôleur programmable extracorporel (2) comprend un récepteur, un émetteur, un synthétiseur de fréquence, un module de traitement numérique, un module d'analyse de données, un module de stockage de données, un afficheur et un processeur ;
le processeur est connecté au module d'analyse de données, au module de stockage de données, à un programme de commande et à l'afficheur ;
le synthétiseur de fréquence est connecté au module de traitement numérique, au récepteur et à l'émetteur ;
le contrôleur programmable extracorporel (2) reçoit des signaux de manière sans fil à travers le récepteur et les transmet au module de traitement numérique, et les signaux sont transmis au processeur à travers un circuit d'interface numérique ;
le processeur transmet les signaux au module d'analyse de données et stocke des résultats d'analyse dans le module de stockage de données, et le processeur transmet les résultats d'analyse au générateur d'impulsion implantable (1) à travers le circuit d'interface numérique, le module de traitement numérique et l'émetteur ;
le module de transmission d'énergie sans fil transmet l'énergie au module de gestion d'alimentation, au récepteur et à l'émetteur ;
le générateur d'impulsion implantable (1) est disposé dans une capsule sous-cutanée créée et le générateur d'impulsion implantable (1) est connecté au muscle extraoculaire (4) par l'intermédiaire de l'électrode (3), et le générateur d'impulsion implantable (1) est configuré pour générer des signaux de stimulation d'impulsions ;
l'électrode (3) comprend un fil (7) et un connecteur à broche (5) et des contacts (6) disposés à deux extrémités du fil, dans laquelle le connecteur à broche (5) est connecté au générateur d'impulsion implantable (1), le fil (7) est disposé dans un tunnel sous-cutané créé et les contacts (6) sont connectés au muscle extraoculaire (4) ;
le contrôleur programmable extracorporel (2) est connecté de manière sans fil par signaux au générateur d'impulsion implantable (1) pour régler des signaux de stimulation d'impulsion pour le générateur d'impulsion implantable (1) ;
dans lequel le générateur d'impulsion implantable (1) est configuré pour détecter des contractions d'un muscle extraoculaire à travers l'électrode (3), et le module de traitement numérique sur le générateur d'impulsion implantable (1) est configuré pour émettre un premier signal de stimulation musculaire extraoculaire à travers l'émetteur en fonction du niveau de contraction du muscle extraoculaire ; l'amplitude et la direction du premier signal de stimulation musculaire extraoculaire sont identiques à l'amplitude et la direction du niveau de contraction du muscle extraoculaire respectivement ; le module de transmission d'énergie sans fil est configuré pour transmettre le premier signal de stimulation musculaire extraoculaire au contrôleur programmable extracorporel (2) ;
le contrôleur programmable extracorporel (2) est également configuré pour analyser le premier signal de stimulation musculaire extraoculaire et émettre un second signal de stimulation musculaire extraoculaire ayant la même amplitude et la direction inverse par rapport au premier signal de stimulation musculaire extraoculaire ;
le module de traitement numérique du générateur d'impulsion implantable (1) est configuré également pour recevoir le second signal de stimulation musculaire extraoculaire émis par le contrôleur programmable extraoculaire et pour transmettre le second signal de stimulation musculaire extraoculaire à un autre muscle extraoculaire ;
le second signal de stimulation musculaire extraoculaire est un signal de stimulation permettant de provoquer une contraction de l'autre muscle extraoculaire vers la direction inverse pour maintenir le globe oculaire fixe.

2. Stimulateur neuromusculaire extraoculaire implantable selon la revendication 1, **caractérisé en ce que**, plusieurs contacts (6) sont disposés sur l'électrode (3), et la connexion entre les contacts (6) et le muscle extraoculaire (4) signifie qu'au moins un contact (6) est connecté au muscle extraoculaire (4) ; les contacts (6) sont connectés au muscle extraoculaire (4) par liaison ou insertion.

3. Stimulateur neuromusculaire extraoculaire implantable selon la revendication 1, **caractérisé en ce que**, le réglage du signal de stimulation d'impulsion comprend : le réglage de l'amplitude d'impulsion, le réglage de la largeur d'impulsion et le réglage de la fréquence d'impulsion.

4. Stimulateur neuromusculaire extraoculaire implantable selon la revendication 3, **caractérisé en ce que**, le réglage de l'amplitude d'impulsion comprend un mode de tension et un mode de courant ; lors du réglage de l'amplitude d'impulsion en mode de tension, une plage de tension est de 0 à 10 V et un pas de réglage est de 1 V ou 0,1 V, et lors du réglage de l'amplitude d'impulsion en mode de courant, une plage de courant est de 0 à 25 mA et un pas de réglage est de 1 mA ou 0.1 mA.

5. Stimulateur neuromusculaire extraoculaire implantable selon la revendication 3, **caractérisé en ce que**, la plage de réglage de la largeur d'impulsion est de 30 à 450 ms, et un pas de réglage est de 10 ms.

6. Stimulateur neuromusculaire extraoculaire implantable selon la revendication 3, **caractérisé en ce que**, la plage de réglage de la fréquence d'impulsion est de 1 à 500 Hz.

7. Procédé de paramétrage pour le stimulateur neuromusculaire extraoculaire implantable selon l'une quelconque des revendications 1 à 6, **caractérisé en ce que**, le réglage du signal de stimulation d'impulsion sur le générateur d'impulsion implantable (1) est effectué par le contrôleur programmable extracorporel (2), comprenant : le réglage de l'amplitude d'impulsion, le réglage de la largeur d'impulsion et le réglage de la fréquence d'impulsion.

8. Procédé de paramétrage pour le stimulateur neuromusculaire extraoculaire implantable selon la revendication 7, **caractérisé en ce que**, le réglage de l'amplitude d'impulsion comprend un mode de tension et un mode de courant ; lors du réglage de l'amplitude d'impulsion en mode de tension, une plage de tension est de 0 à 10 V et un pas de réglage est de 1 V ou 0,1 V, et lors du réglage de l'amplitude d'impulsion en mode de courant, une plage de courant est de 0 à 25 mA et un pas de réglage est de 1 mAou 0.1 mA;
la plage de réglage de la largeur d'impulsion est de 30 à 450 ms, et un pas de réglage est de 10 ms ; et
la plage de réglage de la fréquence d'impulsion est de 1 à 500 Hz.
